# EUROPEAN PATENT SPECIFICATION

(11) **EP 3 305 095 B1**
(45) Date of publication and mention of the grant of the patent: **20.01.2021**
(21) Application number: 17201119.9
(22) Date of filing: 25.02.2014
(51) Int. Cl.: A23J 3/14, C12P 19/32, C12P 13/14, A23L 27/10, A23L 27/24, A23L 27/22, A23L 27/23

(54) **METHOD FOR PREPARING IMP FERMENTED BROTH OR GLUTAMIC ACID FERMENTED BROTH AS RAW MATERIAL FOR PREPARATION OF NATURAL FLAVOR**
VERFAHREN ZUR HERSTELLUNG IMP-FERMENTIERTER BRÜHE ODER GLUTAMINSÄURE-FERMENTIERTER BRÜHE ALS ROHMATERIAL ZUR HERSTELLUNG VON NATÜRLICHEM AROMA
PROCÉDÉ DE PRÉPARATION DE BOUILLON D'IMP FERMENTÉE OU DE BOUILLON D'ACIDE GLUTAMIQUE FERMENTÉ COMME MATIÈRE PREMIÈRE POUR LA PRÉPARATION D'UN ARÔME NATUREL

(30) Priority: 23.07.2013 KR 20130086977
(43) Date of publication of application: 11.04.2018
(62) Divisional of application: 14829615.5
(73) Proprietor: CJ Cheiljedang Corporation, Seoul 100-400 (KR)
(72) Inventor: LEE, Sung Hun, 411-741 Gyeonggi-do (KR); EOM, So Youn, 152-774 Seoul (KR); PARK, Jae Seung, 441-807 Gyeonggi-do (KR); OH, Eun Seon, 463-970 Gyeonggi-do (KR); LEE, Kwang Hee, 462-835 Gyeonggi-do (KR); JANG, Suk Min, 152-857 Seoul (KR); KANG, Dae Ik, 411-750 Gyeonggi-do (KR); CHUNG, Won Dae, 150-894 Seoul (KR)
(74) Representative: Regimbeau

(56) References cited:
- WO-A1-02/051984
- WO-A1-2015/020292
- JP-A- 2000 000 088
- KR-A- 20000 002 408
- KR-A- 20120 076 841
- US-A- 3 385 761
- US-A- 6 066 481
- US-A1- 2001 003 593
- US-A1- 2004 166 200
- US-A1- 2012 178 125
- US-A1- 2012 315 354

## Description

### [Technical Field]

The present invention relates to a method for preparing a natural flavor, and more particularly to a method of preparing a natural flavor using an inosine-5'-monophosphate (IMP) fermented broth or a glutamic acid fermented broth prepared by a two-step fermentation process comprising a first fermentation step for fungal fermentation and a second fermentation step for bacterial fermentation.

### [Background Art]

Amino acids and peptides are used as flavor components. In recent years, natural flavoring materials including amino acids and peptides extracted by fermenting vegetable protein sources, such as soybeans, wheat or corn, with microorganisms, such as fungi, Bacillus, lactic acid bacteria or yeast, and hydrolyzing the fermentation product, have been developed in various ways.

Generally, technology has been developed to improve the extracted taste components (amino acids and peptides) by increasing the degree of hydrolysis of the vegetable protein sources or increase price competitiveness by increasing the yield in terms of mass. However, when only vegetable protein sources are used, there is a disadvantage in that no nucleic acid component is present in the resulting flavoring material, and thus the umami taste intensity of the flavoring material is weak. In addition, flavoring materials are required to contain glutamic acid together with nucleic acids such as inosine monophosphate (IMP) or guanosine monophosphate (GMP) in order to increase the commercial value thereof.

In recent years, in order to overcome the above-described shortcoming of the vegetable protein hydrolysate, natural nucleic acid-containing materials such as yeast extracts have generally been used. However, the yeast extracts may adversely affect the inherent flavor of processed foods due to an off-flavor and off-odor resulting from the peculiar fermented odor thereof, and the nucleic acid content thereof is limited (maximum nucleic acid content: 20%). In addition, mixtures of vegetable protein materials with yeast extracts for use in natural processed foods have low price competitiveness compared to conventional flavoring materials such as monosodium glutamate (MSG), nucleic acid IG, or hydrolyzed vegetable protein (HVP).

Meanwhile, IMP together with GMP is a substance that is widely used as a food flavoring additive. When IMP is used specifically with monosodium glutamate (MSG), it has a significant ability to improve taste. Thus, IMP is a nucleic acid-based flavoring substance that is receiving attention as a flavoring substance.

Methods for fermenting IMP and GMP for use as flavoring substances include a method of degrading yeast RNA, and a method of preparing IMP and GMP by two steps (fermentation and chemical phosphorylation) after preparation of inosine and guanosine. US200416620 disclose fungal fermentation with Asperhillus sp. used in the method of producing seasoning having a umami taste. US2012315354 discloses a process for the preparation of a taste enhancing savory base comprising naturally derived compounds taken in the group consisting of glutamate, IMP and GMP, comprising fermenting a substrate using a microorganism of genus taken in the group consisting of Corynebacterium glutamic, Corynebacterium ammoniagenes, Brevibacterium lactofermentum, Corynebacterium casei, Corynebacterium efficiens and Bacillus subtilis, wherein the substrate is obtained by the enzymatic hydrolysis of a plant material or by the enzymatic hydrolysis of starch. However, in recent years, in various countries, including Europe, standards for natural flavors have been strengthened and regulations have become more severe, and thus flavors which are not made of only natural components but are prepared by performing chemical processes or adding additional components are not recognized as natural flavors. For this reason, natural nucleic acid components should be prepared using a method directly fermenting sugar with bacteria.

Under such circumstances, the present inventors have made extensive efforts to prepare a natural flavor containing no additional component without performing any chemical process, and as a result, have found that, when an IMP or glutamic acid fermented broth produced by a two-step fermentation process comprising a first fermentation step for fungal fermentation and a second fermentation step for bacterial fermentation is used, a variety of effective flavors can be produced even when only the fermented broth is used for a subsequent reaction, thereby completing the present invention.

### [Disclosure]

### [Technical Problem]

It is an object of the present invention to provide a method of preparing a natural flavor containing no additional components, without performing any chemical process, and using an inosine-5'-monophosphate (IMP) fermented broth or a glutamic acid fermented broth prepared by a first fermentation step for fungal fermentation and a second fermentation step for bacterial fermentation.

The present invention also relates to a method for preparing an inosine-5'-monophosphate (IMP) fermented broth or a glutamic acid fermented broth The invention is defined by the appended set of claims and any other aspects or embodiments set forth herein not falling within the scope of the claims are for information only.

### [Technical Solution]

To achieve the above objects, in one aspect, the present invention provides a method for preparing an inosine-5'-monophosphate (IMP) fermented broth or a glutamic acid fermented broth, comprising:(a) fermenting a vegetable protein source with Aspergillus sp. microorganism to obtain a grain fermented broth; and(b) fermenting a part of the grain fermented broth with Corynebacterium sp. or Brevibacterium lactofermentum microorganism to prepare the IMP fermented broth or glutamic acid fermented broth.

In another aspect the invention provides a method for preparing a natural flavor, comprising:(i) preparing the grain fermented broth, and an IMP fermented broth and a glutamic acid fermented broth by the method of claim 1; and(ii) mixing the IMP fermented broth and glutamic acid fermented broth, and wherein the mixing step optionally comprises a step of mixing with the grain fermented broth.

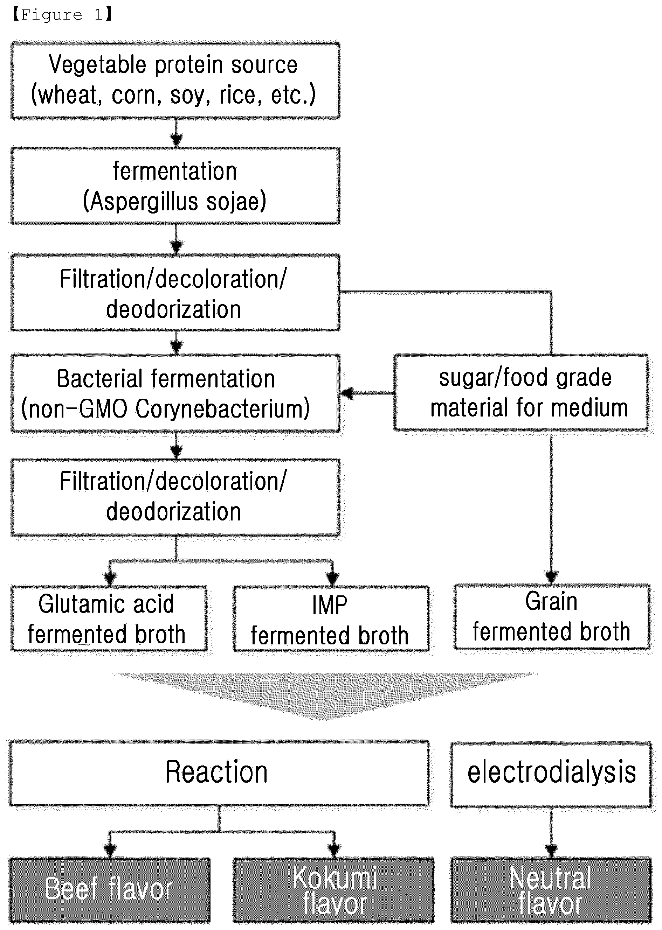

FIG. 1 schematically shows a method for preparing a natural flavor according to the present invention.

A glutamic acid fermented broth, an IMP fermented broth and a grain fermented broth are prepared by a first fermentation step for fungal fermentation and a second fermentation step for bacterial fermentation, and are subjected to a third step wherein a reaction or treatment suitable for each flavor, thereby preparing each natural flavor.

Thus, the present invention is characterized in that a natural IMP fermented broth and a glutamic acid fermented broth that are used as raw materials for preparing natural flavors are prepared by a two-step fermentation process comprising a first fermentation step for fungal fermentation and a second fermentation step for bacterial fermentation.

The first fermentation step for fungal fermentation is a step of producing peptides and amino acids using a protein source. When only the first fermentation step for fungal fermentation is carried out, large amounts of peptides and amino acids can be produced, and thus glutamic acid that may be used as a flavor can be produced. However, there is a disadvantage in that a nucleic acid substance, such as IMP or guanosine monophosphate (GMP), which can give an umami taste, cannot be produced. In addition, there is a problem in that, when a vegetable protein source is used, it is subjected only to a proteolytic process, and thus the peptide and amino acid that can be produced depend on the concentration or content of protein in the protein source. For example, when bean is used, the content of glutamic acid in the produced fermented broth will be less than 10%, and when wheat gluten is used, the content of glutamic acid in the produced fermented broth will be less than 15%.

Meanwhile, the second fermentation step for bacterial fermentation is a step of preparing nucleic acid and glutamic acid fermented broths. When only the second fermentation step for bacterial fermentation is carried out, nucleic acid and glutamic acid can be effectively produced, but there is a shortcoming in that the content of other amino acids and peptides is produced in a concentration of less than 1%, and thus the fermented broth with bacteria cannot be used as a flavor, even though it has a good umami taste. In other words, there is a shortcoming in that, in order to use the fermented broth, obtained only by bacterial fermentation, as a flavor, additional components are required to be added to the fermented broth so that the fermented broth can be applied to foods.

Accordingly, the present inventors have developed a method of preparing a natural flavor, while overcoming the shortcoming of the fungal fermentation process and the bacterial fermentation process, by using only the prepared fermented broth without adding any additional component, and without carrying out any chemical process.

To prepare a nucleic acid and glutamic acid fermented broth using the two-step fermentation process, various mineral salts, amino acids and vitamins together with a carbon source and a nitrogen source are required. Particularly, in the prior art, yeast extract or hydrolyzed vegetable protein (HVP) was used as the nitrogen source, but in this case, there were shortcomings in that the resulting fermented broth had an off-flavor and an off-odor and in that the yield is somewhat low. In addition, the content of flavoring components in the resulting culture broth, as well as the overall flavor, greatly differs depending on various materials that are used for bacterial fermentation. Thus, in the present invention, a grain fermented broth (grain protein hydrolysate), which is obtained by fungal fermentation (first fermentation step) and can contain various amino acids and peptides while serving as a nitrogen source, is used as a substrate for the second fermentation step for bacterial fermentation.

In conventional processes for preparing nucleic acid or MSG using bacteria, special importance was attached to increasing only the concentrations of nucleic acid and MSG in culture broths and the yields thereof. This increase was effective in increasing the umami taste, but in some cases, it adversely affected the flavor of the resulting flavoring substances. However, the various natural flavors prepared by using the 3 steps of the present invention have an advantage in that the flavor can be improved by controlling the concentrations of IMP and glutamic acid, which are umami taste components, as well as various amino acids, saccharides, organic acids and inorganic ions, etc.

Herein, each step of the inventive method for preparing a natural flavor will be described.

Step (a) of the method of the present invention is a step of fermenting a vegetable protein source with fungi to obtain a grain fermented broth.

In step (a), fungal fermentation is performed using a vegetable protein source, thereby obtaining a grain fermented broth that contains various amino acids and peptides and includes components such as glutamine, which can be used as a nitrogen source in the second fermentation step for bacterial fermentation. Specifically, fungi are cultured using a grain material as a substrate to prepare a protease-containing cell culture broth, which is then added to a vegetable protein source, followed by hydrolysis, thereby preparing a grain protein hydrolysate. Then, the grain protein hydrolysate is filtered, and cells are removed therefrom, thereby preparing a grain fermented broth. The grain protein hydrolysate may have a total nitrogen content of 2% (w/v) or more in order to provide a nitrogen source for the second fermentation step for bacterial fermentation.

As the vegetable protein source, any material known in the art may be used in the present invention, as long as it can be fermented with fungi. Examples of the vegetable protein source include, but are not limited to, soybean, corn, rice, wheat gluten, etc. Meanwhile, when wheat gluten is used, it can advantageously increase the yield of bacterial fermentation, because it contains a large amount of glutamine. Thus, wheat gluten may preferably be used as the vegetable protein source.

The fungi that are used in the present invention are *Aspergillus* sp. microorganisms, and more preferably *Aspergillus orizae* or *Aspergillus sojae* microorganisms, but are not limited thereto.

In an example of the present invention, the first fermentation step for fungal fermentation was carried out using *Aspergillus sojae* CJCC_080124P (KCCM11026P) as described in Korean Patent Registration No. 10-1191010 (corresponding to PCT International Publication No. WO 2011-046249).

As used herein, the term "grain fermented broth" refers to a product obtained by fermenting a vegetable protein source (grain) with fungi. The grain fermented broth may be used as a substrate for the second fermentation step for bacterial fermentation and may also be used in a final step of preparing a flavor by subjecting the grain fermented broth to filtration and cell removal processes after fermentation. Thus, the grain fermented broth may be used for two purposes as described above.

Step (b) is a step of fermenting the grain fermented broth, obtained in step (a), with bacteria to prepare an IMP fermented broth and a glutamic acid fermented broth. Specifically, the grain fermented broth obtained in the first fermentation step for fungal fermentation is used as a substrate for bacterial fermentation, and the IMP fermented broth and the glutamic acid fermented broth may be prepared by subjecting the grain fermented broth to bacterial fermentation in a medium supplemented with a carbon source.

The bacterial fermentation can be performed by a general bacterial culture method known in the art, and preferably, it may be composed of three steps: flask culture, scalp-up culture, and main culture. Specifically, flask culture and expansion culture are performed using primary culture medium and secondary culture medium in order to achieve scale-up, after which bacterial fermentation is performed using the grain fermented broth of step (a) as a substrate in main culture medium while continuously supplying additional sugar, thereby obtaining an IMP fermented broth and a glutamic acid fermented broth.

The medium for the bacterial fermentation may comprise a carbon source such as glucose, fructose or the like. Particularly, the medium for preparing the glutamic acid fermented broth may comprise raw sugar as a carbon source. The medium may comprise various mineral salts, vitamins, amino acids and the like, and the composition of the medium may vary depending on the desired fermentation product that is the IMP fermented broth or the glutamic acid fermented broth.

For example, when the IMP fermented broth is to be prepared, the main culture medium may comprise glucose, fructose, magnesium sulfate, phosphoric acid, potassium hydroxide and the grain fermented broth. Preferably, the main culture medium may comprise, based on the total volume of the medium, 4.4-5.2 wt% of glucose, 3.7-4.3 wt% of fructose, 1.3-1.7 wt% of magnesium sulfate, 2.0-2.4 wt% of phosphoric acid, 1.4-1.8 wt% of potassium hydroxide, and 0.5-0.9 wt% of the grain fermented broth. In addition, when the glutamic acid fermented broth is to be prepared, the main culture medium may comprise glucose, fructose, raw sugar, betaine, magnesium sulfate, potassium phosphate and phosphoric acid, and preferably, it may comprise, based on the total weight of the medium, 0.5-0.7 wt% of glucose, 0.9-1.1 wt% of fructose, 4.5-5.5 wt% of raw sugar, 0.005-0.015 wt% of betaine, 0.3-0.5 wt% of magnesium sulfate, 0.8-1.0 wt% of potassium phosphate and 0.2-0.4 wt% of phosphoric acid.

In addition, the medium may, if necessary, comprise small amounts of other components, for example, iron sulfate, manganese sulfate, copper sulfate, zinc sulfate, CAPA, NCA (nicotinamide), biotin, calcium chloride, thiamine, vitamin C, and the like. Typical examples of each of the media comprising these components are shown in Tables 5 to 8 and 9 to 12.

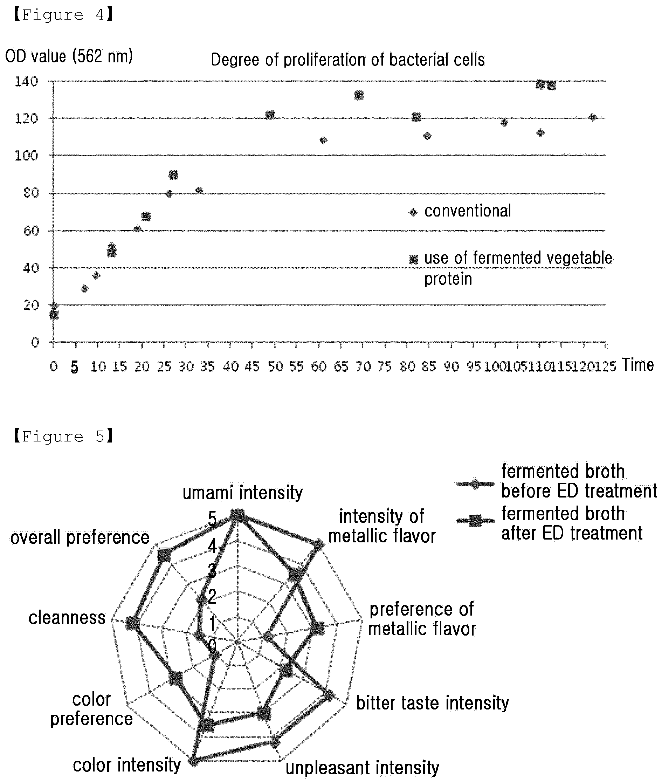

As a result of the fungal fermentation in step (a), the vegetable protein source is decomposed into amino acids and peptides, and inorganic ions, such as calcium, magnesium and phosphate ions, and vitamins are eluted from the protein source. The amino acids produced by the fungal fermentation include glutamine, cysteine, methionine, valine, leucine, isoleucine and the like, and may be used as a nitrogen source in the bacterial fermentation in step (b). Particularly, a high concentration of glutamine is an essential component for purine biosynthesis and can act as a major promoter for production of high contents of IMP and glutamic acid. In addition, the eluted inorganic ions and vitamins can be helpful in the growth of bacterial cells in the bacterial fermentation. In an example of the present invention, it was shown that, when the product obtained by the fungal fermentation was used as a nutrient source, the proliferation and growth of bacterial cells became faster (FIG. 4). This further demonstrates the advantage of the two-step process of the present invention.

The bacteria that are used in the present invention are *Corynebacterium* sp. microorganisms. Most preferably, *Corynebacterium ammoniagenes* may be used to prepare the IMP fermented broth, and *Corynebacterium glutamicum* may be used to prepare the glutamic acid fermented broth. In addition, as the bacteria, various bacteria, disclosed in previous patent documents and known to have the capability to produce IMP and glutamic acid, may be used.

In an example of the present invention, to prepare an IMP fermented broth, *Corynebacterium ammoniagenes* CJIP009 (KCCM-10226) described in Korean Patent Registration No. 10-0397321 (corresponding to WO International Patent Publication No. WO2002-051984) was used, and to prepare a glutamic acid fermented broth, *Corynebacterium glutamicum (Brevibacterium lactofermentum)* CJ971010 (KFCC 11039) described in Korean Patent Registration No. 10-0264740 (published as KR 2000 0002408) was used.

The fermented broth obtained by bacterial fermentation in step (b) has a solid content of about 150 g/L. The present invention is characterized in that the IMP and/or glutamic acid fermented broth obtained by the bacterial fermentation is used in a natural flavor preparation process without adding any additional component thereto, the fermented broth should contain large amounts of IMP and glutamic acid that are the desired components.

Thus, the solid in the IMP fermented broth prepared by the first fermentation step for fungal fermentation and the second fermentation step for bacterial fermentation may preferably have an IMP content of 30% or more, and more preferably 50% or more, but is not limited thereto. Accordingly, the concentration of IMP in the IMP fermented broth may preferably be 50 g/L to 150 g/L, and more preferably 70 g/L to 130 g/L.

Further, because the product produced by the first fermentation step for fungal fermentation contains amino acids, the glutamic acid can be obtained at high concentration and high yield compared to those of IMP. The solid in the prepared glutamic acid fermented broth may preferably have a glutamic acid content of 50% or more, and more preferably 60% or more, but is not limited thereto. Thus, the concentration of glutamic acid in the glutamic acid fermented broth may preferably be 75 g/L to 150 g/L, and more preferably 90 g/L to 130 g/L.

Due to high contents of IMP and glutamic acid in the fermented broth as described above, when the fermented broth is powdered by a process such as drying, it can be suitably added to food.

As used herein, the term "flavor" refers to a substance that is added to improve the flavor of food. The flavor can be classified according to its component into various flavors. Specific examples of the flavor include a neutral flavor, a beef flavor, a chicken flavor, a pork flavor, and a kokumi flavor. Each of the flavors can be prepared using the IMP fermented broth and glutamic acid fermented broth prepared by the two-step fermentation process of the present invention. Among them, the term "neutral flavor" refers to a flavor that gives a mild and clean taste by maximizing an umami taste and minimizing other flavors. For example, it has been reported that oils such as canola oil or grape seed oil has neutral flavor.

Because the fermented broth of the present invention is characterized in that it is used in a natural flavor preparation process without adding any additional components and without subjecting it to an additional chemical process such as purification, all medium components should be food grade materials in order for the fermented broth to be included directly in food, for example, processed food. Thus, all medium components that are added during fermentation are preferably food grade materials. In an example of the present invention, in order to use food grade materials as medium components, β-alanine was replaced with calcium panthothenate (CAPA), and nevertheless, it was shown that an IMP fermented broth having an IMP concentration of 70 g/L or more could be prepared. Thus, the medium for bacterial fermentation according to the present invention may preferably contain CAPA.

Each of the IMP fermented broth and the glutamic acid fermented broth can be prepared by the two-step fermentation process comprising steps (a) and (b), and the prepared fermented broth can finally be subjected to a third step for reaction and can be used in a process of preparing various flavors. Based on the IMP fermented broth and the glutamic acid fermented broth, various natural flavors, for example, neutral flavors, and flavors for beef, chicken, pork, kokumi and the like, can be prepared by using different raw materials, or slightly changing the medium composition, or controlling process conditions, including temperature, pressure and time, in the process of mixing the fermented broths, or a reaction or electrodialysis process disclosed below. Among the prepared natural flavors, a flavor suitable for the purpose of each food may be added to the food to give the optimum taste.

According to specific embodiment of the present invention, the method for preparing an inosine-5'-monophosphate (IMP) fermented broth or a glutamic acid fermented broth, comprises:
(a) fermenting a vegetable protein source with Aspergillus sp. microorganism to obtain a grain fermented broth; and
(b) fermenting a part of the grain fermented broth with Corynebacterium sp. or Brevibacterium sp. microorganism.

Particularly, the IMP fermented broth or the glutamic acid fermented broth are raw materials for preparing a natural flavor.

Moreover, in the method for preparing an inosine-5'-monophosphate (IMP) fermented broth or a glutamic acid fermented broth the vegetable protein source is selected from the group consisting of soybean, corn, rice, wheat, and wheat gluten.

In particular embodiment of this method, the *Aspergillus sp.* microorganism is *Aspergillus sojae.*

In another particular embodiment of this method, the Corynebacterium sp. microorganism is *Corynebacterium ammoniagenes* or *Corynebacterium glutamicum.*

In still another particular embodiment of this method, the *Brevibacterium sp.* microorganism is *Brevibacterium lactofermentum.*

In the method for preparing an inosine-5'-monophosphate (IMP) fermented broth or a glutamic acid fermented broth according to the invention, a medium composition for bacterial fermentation for preparing the IMP fermented broth or the glutamic acid fermented broth comprises a food grade material, and optionally comprises calcium panthothenate (CAPA).

In one embodiment, a medium for bacterial fermentation for preparing the IMP fermented broth comprises glucose, fructose, magnesium sulfate, phosphoric acid, potassium hydroxide and a grain fermented broth.

In another embodiment, a medium for bacterial fermentation for preparing the glutamic acid fermented broth comprises glucose, fructose, raw sugar, betaine, magnesium sulfate, potassium phosphate and phosphoric acid.

The method for preparing an inosine-5'-monophosphate (IMP) fermented broth or a glutamic acid fermented broth according to the invention also comprises a carbon source in a medium for bacterial fermentation for preparing the glutamic acid fermented broth is raw sugar.

According to specific embodiment of said method, the concentration of IMP in the prepared IMP fermented broth is 50 g/L to 150 g/L, or the content of IMP in solids in the prepared IMP fermented broth is 30 wt% or more and/or the concentration of glutamic acid in the prepared glutamic acid fermented broth is 75 g/L to 150 g/L, or the content of glutamic acid in solids in the prepared glutamic acid fermented broth is 50 wt% or more.

Step (c) of the method according to the invention is a step of electrodialyzing the IMP fermented broth in order to have the physical properties suitable for neutral flavors. Because the IMP fermented broth includes sulfuric acid, ammonia, and various metallic ions such as Mg and Mn due to its fermentation process, the IMP fermented broth has off-flavor and off-odor not suitable for a neutral flavor intended to have a clean taste. Therefore, the electrodialysis step removes off-flavor and off-odor to eliminate unpleasant or bitter taste from the neutral flavor and enables the neutral flavor to have cleaner and milder taste and can result in having the physical properties suitable for neutral flavors. Additionally, but not essentially, the electrodialysis step also can enable the glutamic acid fermented broth which has relatively less off-flavor and off-odor compared to the IMP fermented broth to have a cleaner taste.

In an example of the present invention, a neutral flavor was prepared using the electrodialysis process, and the sensory attributes of the prepared flavor were compared with those of a sample prepared without the electrodialysis process. As a result, it was shown that the intensity of metallic flavor, bitter taste, unpleasant intensity and color darkness of the prepared flavor were all reduced, and thus the prepared flavor had a clean and mild flavor and overall preference for the flavor increased (FIG. 5).

As used herein, the term "electrodialysis (ED)" refers to a process of separating ionic components from a solution. It is theoretically based on the mass transfer principle in which ionic components in a solution are selectively passed through a cation exchange resin membrane and an anion exchange resin membrane by a voltage applied to an electric field. It is a membrane process that is most frequently used together with reverse osmosis and ultrafiltration processes. Because the electrodialysis process is not interpreted as a chemical process, it can be used in the natural flavor preparation process according to the purpose of the present invention. For the purpose of the present invention, ions in the fermented broth, for example, metallic ions, particularly monovalent and divalent cations and anions, can be removed by the electrodialysis process, thereby removing off-odor.

Treating with activated carbon is a well-known process for removing off-flavor and off-odor through decolorization and deodorization. However, treatment with activated carbon has drawbacks in that activated carbon can result in decolorization and deodorization through physical absorption, but cannot filter out all ionic components. Therefore, in the present invention, natural neutral flavors having a more clean and mild taste can be prepared by the electrodialysis process, thereby removing off-flavor and off-odor.

Meanwhile, the step (c) may further comprise a step of treating the fermented broth with activated carbon before the electrodialysis process. In addition, the electrodialysis process can be carried out after centrifuging or filtering the fermented broth treated with activated carbon. After the fermented broth is treated with activated carbon, it may further be treated with diatomaceous earth as a filtration aid. Moreover, before the fermented broth is treated with activated carbon, it may be subjected to a pretreatment process of heating the fermented broth to induce cell lysis, in order to increase the yield of the filtration process that is subsequently carried out. The heating process may preferably be carried out at 70∼90 °C, and the heating time may preferably be 15 minutes or longer, and more preferably 15-60 minutes.

According to a specific embodiment, the method for preparing an inosine-5'-monophosphate (IMP) fermented broth or a glutamic acid fermented broth further comprises a step (c) of treating the fermented broth, obtained by the bacterial fermentation, with activated carbon; and optionally further comprising:
(i) after treating the fermented broth with activated carbon, centrifuging or filtering the treated fermented broth, or
(ii) before treating the fermented broth with activated carbon, heating the fermented broth at a temperature of 70 to 90 °C for 15 to 60 minutes to induce cell lysis.

Step (d) of the method of the invention is a step of mixing the glutamic acid fermented broth and IMP fermented broth obtained from the prior steps in order to prepare natural neutral flavors. In addition, the step (d) may further comprise a step of mixing with the grain fermented broth.

In step (d), natural neutral flavors can be finally prepared by mixing the IMP fermented broth and the glutamic acid fermented broth through the preparation method of the present invention. Herein the mixing ratio of the IMP fermented broth and the glutamic acid fermented broth may preferably be 0.2:1 to 5:1, more preferably 0.5:1 to 2:1 and most preferably 1:1, but is not limited thereto. Moreover, the mixed fermented broth can further be mixed with the grain fermented broth.

The mixing step of the present invention may further comprise a step of concentrating the fermented broth and drying the concentrate to prepare powder. The step of preparing the fermented broth into powder may be performed before or after mixing the fermented broths, and can preferably be performed before mixing the fermented broths. The drying can preferably be achieved by spray drying or vacuum drying. The fermented broth can be finally prepared into powder or paste which is suitable for addition to food.

In one aspect the invention further relates to method for preparing a natural flavor, comprising:
(i) preparing the grain fermented broth, and an IMP fermented broth and a glutamic acid fermented broth by the method for preparing an inosine-5'-monophosphate (IMP) fermented broth or a glutamic acid fermented broth of the invention; and
(ii) mixing the IMP fermented broth and glutamic acid fermented broth,

Wherein the mixing step optionally comprises a step of mixing with the grain fermented broth.

According to one embodiment of said method, the natural flavor is prepared using only the grain fermented broth, IMP fermented broth and glutamic acid fermented broth without adding any additional component thereto and/or without subjecting the fermented broth to an additional chemical process.

According to another embodiment, said method further comprises, before step (b), electrodialyzing the IMP fermented broth or the glutamic acid fermented broth.

In another aspect, the present invention provides a natural neutral flavor prepared by the preparation method of the present invention. In still another aspect, the present invention also provides a food composition comprising the natural neutral flavor.

The natural neutral flavor prepared by the preparation method of the present invention has a clean and mild taste, and therefore maybe added to a suitable food to maximize the taste of the food and may also be applied to animal food. For example, the natural neutral flavor may be added to snack seasonings, soup stocks, commercial flavors, dressings and the like to produce a clean taste.

### [Advantageous Effects]

The natural flavors prepared according to the method of the present invention are prepared using natural raw materials, and thus are harmless and safe for use in the human body, and may be added to food to produce clean and mild tastes, and improve the flavor of the food.

### [Description of Drawings]

FIG. 1 is a schematic view showing an overall process for preparing a natural flavor.
FIG. 2 shows a process of acid-hydrolyzing AMP in order to replace adenine with a natural food grade material according to the present invention.
FIG. 3 shows a metabolic pathway for deriving CAPA to replace β-alanine with a natural food grade material.
FIG. 4 shows the degree of proliferation of bacterial cells in the case in which a vegetable protein degraded by fungal fermentation according to the present invention was added as a nutrient source and in the case in which the vegetable protein was not added.
FIG. 5 shows the results of evaluating sensory attributes before and after an electrodialysis process.
FIG. 6 shows the results of comparison of sensory attributes between the neutral flavor of the present invention and a yeast extract.

### [Mode for Invention]

Hereinafter, the present invention will be described in further detail with reference to examples. However, it will be obvious to those skilled in the art that these examples are for illustrative purposes only and are not intended to limit the scope of the present invention.

### Example 1: Fungal fermentation of vegetable protein source

For primary fungal fermentation, a substrate including grain materials such as soybean, corn, rice or wheat was cultured using *Aspergillus* sp. microorganisms as a fungal strain at a temperature of 20∼35 °C for 24-72 hours, thereby preparing a fungal culture broth containing a high concentration of protease. Then, a vegetable protein source such as soybean, corn, rice or wheat was mixed with water to a high concentration of 25-35% and sterilized, and the above-prepared fungal culture broth containing a high concentration of protease was added to the sterilized vegetable protein source in a salt-free state to hydrolyze the vegetable protein source. Herein, the fungal culture broth was added in an amount of 10-100% based on the sterilized substrate solution, and the substrate was degraded at 40∼50 °C for 48-96 hours to prepare a grain protein hydrolysate.

Specifically, flask culture and expansion culture were performed using *Aspergillus sojae* CJCC_080124P (KCCM11026P) as described in Korean Patent Registration No. 10-1191010 (corresponding to PCT International Publication No. WO 2011-046249), and then a fungal culture broth was prepared using defatted soybean, and wheat gluten as a substrate was hydrolyzed, thereby preparing a grain protein hydrolysate. More specifically, 200 ml of a primary culture was dispensed in a 1 L flask and sterilized, and 200 ml of fungal cells were inoculated in the flask at a density of 1.7×10¹⁰ fungal cells/400 ml and cultured at 30 °C and 100 rpm for 7 hours. Then, a secondary culture was added to a 250 L fermentor and sterilized, and 600 ml of the primary culture broth was inoculated therein and cultured at 30 °C and 70 rpm for 24 hours. Next, a main culture medium was added to a 5-ton preparatory tank and heated at 90 °C for 30 minutes, after it was transferred into a 8-ton fermentor, and 100 L of water and 2 L of a defoaming agent were added thereto. Then, 144 L of the secondary culture broth was inoculated in the main culture medium, and then cultured at 30 °C and 700 rpm for 48 hours, thereby preparing a fungal culture broth. Finally, a raw material for substrate hydrolysis was added to a 5-ton preparatory tank and heated at 55 °C for 1 hour, after which it was transferred into a 20-ton fermentor, and 100 L of water and 2 L of a defoaming agent were added thereto, followed by sterilization. Then, 5760 L of the fungal culture broth was inoculated in the substrate and cultured at 45 °C and 30 rpm for 96 hours, thereby preparing a grain protein hydrolysate. The compositions of the media used in the preparation of the grain protein hydrolysate are shown in Tables 1 to 4 below.

**[Table 1]**

| Primary culture for fungal fermentation | | | |
|---|---|---|---|
| Medium component | Unit | Amount | % |
| Glucose | g | 6 | 3 |
| KH₂PO₄ | g | 6 | 3 |
| Yeast extract | g | 6 | 3 |
| Water | ml | 182 | |

**[Table 2]**

| Secondary culture for fungal fermentation | | | |
|---|---|---|---|
| Medium component | Unit | Amount | % |
| Glucose | kg | 1.44 | 1 |
| KH₂PO₄ | kg | 1.44 | 1 |
| Yeast extract | kg | 1.44 | 1 |
| Water | kg | 138.6 | |

**[Table 3]**

| Main culture medium for fungal fermentation | | | |
|---|---|---|---|
| Medium component | Unit | Amount | % |
| Defatted soybean | kg | 103.68 | 1.9 |
| KH₂PO₄ | kg | 57.6 | 1 |
| Water | kg | 5352 | |

**[Table 4]**

| Raw material for substrate hydrolysis | | |
|---|---|---|
| Raw material | Unit | Amount |
| Wheat gluten | kg | 2650 |
| Water | L | 7500 |

The grain protein hydrolysate prepared as described above was filtered through a filter press to remove fungal cells, thereby preparing a grain fermented broth having a total nitrogen content of 2%(w/v) or more and a degree of hydrolysis of 50% or more. Meanwhile, the grain fermented broth was prepared as a medium for secondary bacterial fermentation.

### Example 2: Bacterial fermentation

To prepare an IMP fermented broth and a glutamic acid fermented broth using the grain fermented broth, a carbon source 21 such as glucose or fructose, mineral salts such as Fe, Mg, Mn and Zn, and vitamins were added to the grain fermented broth prepared in Example 1, followed by sterilization, thereby preparing media for bacterial fermentation. The media for preparing an IMP fermented broth and a glutamic acid fermented broth were prepared, and for each of the fermented broths, media for flask culture (primary culture), expansion culture (secondary culture) and main culture were prepared.

### 2-1: Preparation of IMP fermented broth

Using *Corynebacterium ammoniagenes* CJIP009 (KCCM-10226) described in Korean Patent Registration No. 10-0397321 (corresponding to WO International Patent Publication No. WO2002-051984), an IMP fermented broth having an IMP concentration of 70 g/L or more was prepared.

Specifically, 50 ml of a primary culture adjusted to a pH of 7.2 using NaOH was dispensed in a 500-ml flask and sterilized at 121 °C for 15 minutes, after the bacterial strain was inoculated into the medium and cultured at 32 °C and 200 rpm for 22-28 hours. Then, 2.1 L of a secondary culture was dispensed in a 5-L jar, sterilized and cooled, after which 300 ml of the primary culture broth was inoculated therein and cultured in 2 L of air at 32 °C and 900 rpm for 27-30 hours while adjusting the pH to 7.2. Next, 8.5 L of a main culture medium was dispensed in a 30-L jar, sterilized and cooled, after which 1500 ml of the secondary culture broth was inoculated therein and in 5 L of air at 32 °C and 400 rpm for 5-6 days while adjusting the pH to 7.2 and supplying an additional sugar comprising a mixture of glucose and fructose at any time. After supply of the final additional sugar, the culture was performed for 7 hours or longer so that the sugar could be completely consumed. The compositions of the media used in the preparation in the IMP fermented broth are shown in Tables 5 to 8 below.

**[Table 5]**

| Primary culture for preparation of IMP fermented broth | | | |
|---|---|---|---|
| Medium component | Unit | Amount | % |
| Glucose | g | 10 | 1 |
| Grain fermented broth | g | 35 | 3.5 |
| NaCl | g | 2.5 | 0.25 |
| Volume of medium | ml | 1000 | |

**[Table 6]**

| Secondary culture for preparation of IMP fermented b roth | | | |
|---|---|---|---|
| Medium component | Unit | Amount | % |
| Glucose | g | 78.93 | 8 |
| Grain fermented broth | g | 21.43 | 2 |
| H₃PO₄ (85%) | g | 2.14 | 0.21 |
| KOH (85%) | g | 1.77 | 0.18 |
| MgSO₄7H₂O | g | 1.43 | 0.14 |
| (NH₄)₂SO₄ | g | 7.14 | 0.71 |
| L-cysteine HCl | mg | 28.6 | |
| CAPA | mg | 21.4 | |
| Biotin | mg | 0.09 | |
| Thiamine | mg | 7.1 | |
| FeSO₄7H₂O | mg | 28.6 | |
| MnSO₄7H₂O | mg | 14.3 | |
| ZnSO₄7H₂O | mg | 14.3 | |
| Volume of medium | ml | 1000 | |

**[Table 7]**

| Main medium for preparation of IMP fermented broth | | | |
|---|---|---|---|
| Medium component | Unit | Amount | % |
| H₃PO₄ (85%) | g | 22.21 | 2.2 |
| KOH (85%) | g | 15.86 | 1.6 |
| Grain fermented broth | g | 7 | 0.7 |
| CuSO₄5H₂O | mg | 12.2 | |
| FeSO₄7H₂O | mg | 36.5 | |
| ZnSO₄7H₂O | mg | 36.5 | |
| MnSO₄7H₂O | mg | 109.6 | |
| L-cysteine HCl | mg | 40.4 | |
| CAPA | mg | 34.7 | |
| NCA | mg | 34.9 | |
| Biotin | mg | 0.15 | |
| CaCl₂2H₂O | mg | 182.6 | |
| Thiamine | mg | 23.5 | |
| MgSO₄7H₂O | g | 14.61 | 1.5 |
| Glucose | g | 48.21 | 4.8 |
| Fructose | g | 40.22 | 4 |
| Volume of medium | ml | 1000 | |

**[Table 8]**

| Additional sugar for preparation of IMP fermented br oth | | | |
|---|---|---|---|
| Medium component | Unit | Amount | % |
| Glucose | g | 358.97 | 35.9 |
| Fructose | g | 299.58 | 30 |
| NCA | mg | 772 | 0.08 |
| CAPA | mg | 936.6 | 0.09 |
| H₃PO₄ (85%) | g | 55.68 | 5.6 |
| NaOH (100%) | g | 13.5 | 1.4 |
| Volume of medium | ml | 1000 | |

### 2-2: Preparation of glutamic acid fermented broth

Using *Corynebacterium glutamicum* (*Brevibacterium lactofermentum*) CJ971010 (KFCC 11039) described in Korean Patent Registration No. 10-0264740 (published as KR2000 0002408), a glutamic acid fermented broth having a glutamic acid concentration of 90 g/L or more was prepared.

Specifically, 30 ml of a primary culture was dispensed in a 250-ml flask and sterilized, and the bacterial strain was inoculated in the medium and cultured for 5-8 hours. Then, 1.4 L of a secondary culture was dispensed in a 5-L jar, sterilized and cooled, and then 20 ml of the primary culture broth was inoculated therein and cultured for 20-28 hours. Then, 9.2 L of a main culture medium was dispensed in a 30-L jar, sterilized and cooled, after which 800 ml of the secondary culture broth was inoculated therein and cultured for 36-45 hours while supplying an additional sugar at any time.

After supply of the final additional sugar, the culture was performed for 7 hours or longer so that the sugar could be completely consumed. The compositions of the media used in the preparation in the glutamic acid fermented broth are shown in Tables 9 to 12 below.

**[Table 9]**

| | | | |
|---|---|---|---|
| Primary culture for preparation of glutamic acid fermented broth | | | |

| Medium component | Unit | Amount | % |
|---|---|---|---|
| Raw sugar (98.5%) | g | 20.3 | 2 |
| Grain fermented broth | g | 13 | 1.3 |
| (NH₄)₂SO₄ | g | 13.72 | 1.4 |
| KH₂PO₄ | g | 5.2 | 0.5 |
| K₂HPO₄ | g | 10.7 | 1 |
| MgSO₄7H₂O | g | 0.5 | 0.05 |
| d-biotin | mg | 1.8 | |
| Thiamine-HCl | mg | 9 | |
| CAPA | mg | 9 | |
| Niacinamide (nicotinamide) | mg | 60 | |
| Volume of medium | ml | 1000 | |

**[Table 10]**

| | | | |
|---|---|---|---|
| Secondary culture for preparation of glutamic acid fermented broth | | | |

| Medium component | Unit | Amount | % |
|---|---|---|---|
| Glucose | g | 81.79 | 8.2 |
| Fructose | g | 23.14 | 2.3 |
| H₃PO₄ (85%) | g | 2.73 | 0.27 |
| MgSO₄7H₂O | g | 1.03 | 0.1 |
| Betaine | g | 0.14 | 0.01 |
| DL-Meth | g | 0.15 | 0.01 |
| FeSO₄7H₂O | mg | 18.6 | |
| MnSO₄7H₂O | mg | 18.6 | |
| CuSO₄5H₂O | mg | 3.6 | |
| ZnSO₄7H₂O | mg | 3.6 | |
| Thiamine-HCl | mg | 7.1 | |
| Vitamin C (ascorbic acid) | mg | 7.1 | |
| Succinate | mg | 1.1 | |
| d-biotin | mg | 17.4 | |
| Vitamin B12 (cyanocobalamin) | mg | 0.057 | |
| Grain fermented broth | g | 42.86 | 4.3 |
| Volume of medium | ml | 1000 | |

**[Table 11]**

| | | | |
|---|---|---|---|
| Main culture medium for preparation of glutamic acid fermented broth | | | |

| Medium component | Unit | Amount | % |
|---|---|---|---|
| Glucose | g | 5.86 | 0.6 |
| Fructose | g | 10.48 | 1 |
| Raw sugar | g | 50 | 5 |
| Betaine | g | 0.97 | 0.1 |
| MgSO₄7H₂O | g | 3.69 | 0.4 |
| K₂HPO₄ | g | 9 | 0.9 |
| H₃PO₄ (85%) | g | 3.1 | 0.3 |
| FeSO₄7H₂O | mg | 22.9 | |
| MnSO₄7H₂O | mg | 22.9 | |
| CuSO₄5H₂O | mg | 2.3 | |
| ZnSO₄7H₂O | mg | 2.3 | |
| CAPA | mg | 2.9 | |
| NCA | mg | 2.9 | |
| Biotin | mg | 0.29 | |
| CaCl₂2H₂O | mg | 71.4 | |
| Thiamine-HCl | mg | 0.4 | |
| Vitamin C (ascorbic acid) | mg | 1.7 | |
| Volume of medium | ml | 1000 | |

**[Table 12]**

| | | | |
|---|---|---|---|
| Additional sugar for preparation of glutamic acid fermented broth | | | |

| Medium component | Unit | Amount | % |
|---|---|---|---|
| Glucose | g | 192.3 | 19.2 |
| Fructose | g | 54.33 | 5.4 |
| Raw sugar | g | 147.33 | 14.7 |
| Betaine | g | 0.67 | 0.07 |
| Grain fermented broth | g | 4 | 0.4 |
| CAPA | mg | 15.87 | |
| NCA | mg | 15.87 | |
| Volume of medium | ml | 1000 | |

In order to prepare the IMP fermented broth using the media for preparation of the IMP fermented broth and use the IMP fermented broth as a flavor for addition to food, several medium 27 components should be replaced with food grade materials so as to satisfy the requirement of a food additive. Thus, adenine and β-alanine necessary for cell growth were replaced with food grade materials.

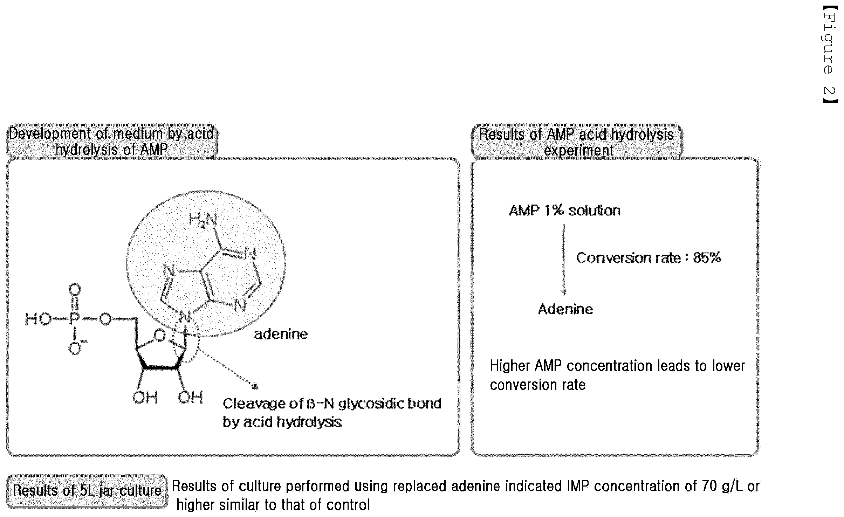

First, adenine was prepared by acid-hydrolyzing AMP to cleave a β-N-glycosidic bond with ribose to thereby liberate adenine. It was shown that, when culture was performed using the adenine prepared as described above, an IMP fermented broth having an IMP concentration of 70 g/L or higher could be prepared (FIG. 2).

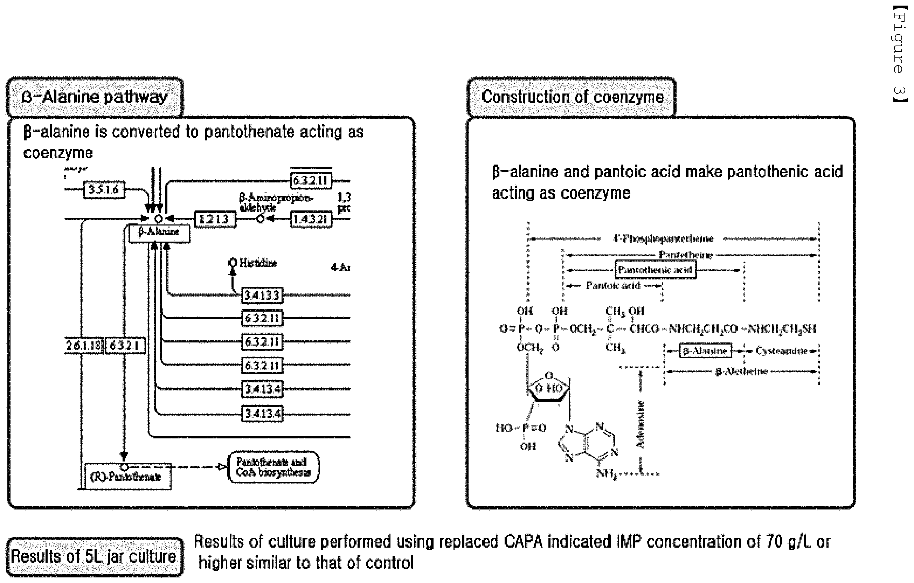

Then, as a result of examining the metabolic pathway of *Corynebacterium ammoniagenes,* it was expected that β-alanine would be replaced with calcium panthothenate (CAPA). Thus, culture was performed using CAPA in place of β-alanine, and as a result, it was shown that an IMP fermented broth having an IMP concentration of 70 g/L or higher could be prepared (FIG. 3).

### Example 3: Examination of cell growth rate in case in which fungal fermentation product is used in bacterial fermentation

In order to confirm the advantage of the case in which the first fermentation step for fungal fermentation and the second fermentation step for bacterial fermentation are continuously performed, the growth rate and degree of proliferation of bacterial cells in the case in which the vegetable protein degraded by the first fungal fermentation step is added as a nutrient source and in the case in which the vegetable protein is not added were examined.

As a result, as can be seen in FIG. 4, in the case in which the vegetable protein degraded by the first fungal fermentation step was added as a nutrient source, the bacterial cells proliferated at a high rate and in a large amount compared to those in the case in which the vegetable protein was not added (FIG. 4).

### Example 4: Examination of the change in flavor characteristic of glutamic acid fermented broth according to the kind of carbon source

Carbon sources that may be used in glutamic acid fermentation include glucose, fructose, cane molasses, raw sugar, etc. Although some other medium components vary depending on the kind of carbon source, the glutamic acid concentration of the resulting fermented broth does not significantly depend on the kind of carbon source. However, the flavor of the fermented broth significantly changes depending on the kind of carbon source, and to develop a neutral flavor, the resulting fermented broth preferably has a cleaner flavor. Cane molasses is an excellent medium component in terms of microbial fermentation due to various inorganic ions contained therein, but has a shortcoming in that the value of use thereof as a neutral flavor is low, because the color of the medium itself is too dark and a flavor caused by caramelization remains in the resulting culture broth.

Thus, raw sugar was used in place of cane molasses in the preparation of a glutamic acid fermented broth for preparing a neutral flavor, and the resulting change in the flavor characteristic of the culture broth was examined. Herein, while cane molasses was replaced with raw sugar, major inorganic ions and vitamins contained in cane molasses were additionally added to the medium. As a result, it could be seen that, when cane molasses was replaced with raw sugar, off-flavor was removed, and thus a flavor characteristic suitable for preparing a neutral flavor appeared (Table 13). In addition, the glutamic acid fermented broth prepared using raw sugar also contained a high concentration (96 g/L or more) of glutamic acid, which is a glutamic acid concentration suitable for preparing a neutral flavor.

**[Table 13]**

| | | | |
|---|---|---|---|
| Change in flavor characteristic of culture broth as a function of ratio of cane molasses and raw sugar | | | |

| | Cane molasses (100%) | Cane molasses + raw sugar (5:5) | Raw sugar (100%) |
|---|---|---|---|
| Neutrality | - | + | ++ |
| Off-flavor | + | - | -- |

### Example 5: Preparation of neutral flavor

Using the glutamic acid fermented broth and IMP fermented broth prepared in Example 2, a neutral flavor was prepared.

Specifically, in order to increase the yield of a filtration process to be performed subsequently, each of the fermented broths was heated at 70∼90 °C for 15 minutes or more to induce cell lysis. This pretreatment process can increase the filtration yield to 85% or higher. Then, to remove off-flavor and off-odor from the glutamic acid fermented broth and the IMP fermented broth, activated carbon was added in an amount of 1-3% (w/v) based on the volume of the fermented broth, which was then treated with the activated carbon at 50∼70 °C for 2-24 hours. After treatment with activated carbon, 1-3% (w/v) of diatomaceous earth as a filtration aid was added to the fermented broth, which was then filtered through a filter press.

The filtrate treated with activated carbon was subjected to electrodialysis to remove monovalent and divalent ions, including Ca, Fe, K, Mg and NH₄ ions, to thereby remove an off-flavor resulting from ammonia and metallic ions, thereby preparing a neutral flavor.

When the fermented broth was subjected to electrodialysis at a flux of 5-20 L/hr.m², monovalent and divalent ions, such as Ca, Fe, K, Mg and Mn ions, were reduced by 30-60%. The contents of the components of the fermented broth before and after performing the electrodialysis process are shown in Table 14 below. As can be seen therein, the contents of inorganic ions in the fermented broth decreased after the electrodialysis process, while the fermented flavor had a cleaner and milder flavor.

Finally, refined salt and maltodextrin were added to the filtrate obtained by the culture and filtration processes as described above, and the mixture was powdered by spray drying, thereby preparing a natural flavor of the present invention.

**[Table 14]**

| | IMP (g/L) | Ca | Cu | Fe | K | Mg | Mn | Na | Zn | Cl- | SO₄ | PO4 | NH₄ |
|---|---|---|---|---|---|---|---|---|---|---|---|---|---|
| Before ED treatment | 69.6 | 87 | <0.1 | 1 | 98 | 77 | 0.8 | 251 | <0.1 | 64 | 1100 | 13700 | 6230 |
| After ED treatment | 63.7 | 35 | <0.1 | 0.3 | 73 | 59 | 0.2 | 234 | <0.1 | <0.1 | 390 | 5720 | 3350 |

### Example 6: Evaluation of sensory attributes caused by electrodialysis process

In order to verify the flavor of the inventive neutral flavor prepared as described above, evaluation of sensory attributes was performed.

Each of a sample from which inorganic ions were removed by electrodialysis, and a sample not subjected to electrodialysis, was powdered, and then the sensory attributes of the samples were evaluated by panels. The panels were professionally trained so that they could be sensitive to sensory attributes, and the panels were provided with the same concentration of the samples so as to evaluate the sensory attributes and intensity of each sample.

As a result, as shown in FIG. 5, the intensity of umami taste did not substantially differ between before and after the electrodialysis process, but in the case of the fermented broth before the electrodialysis process, metallic flavor, bitter taste, unpleasantness and color darkness, which act as negative factors in sensory attributes, were all high, and thus the cleanness and overall preference of the fermented broth were low. However, in the case of the fermented broth subjected to the electrodialysis process, the intensity of metallic flavor, bitter taste, unpleasant intensity, and color darkness were all low, and thus the preferences to flavor and color were all high and the cleanness and the overall preference were high. Accordingly, it could be seen that, when the electrodialysis process was performed, a neutral flavor could be prepared having a clear flavor and for which there was an improved preference, as compared to those before the electrodialysis process.

### Example 7: Comparison of sensory attributes with yeast extract

In order to examine the flavoring effect of the neutral flavor of the present invention, the sensory attributes thereof were compared with those of a yeast extract that is one of natural flavors that have recently been most widely and variously used.

In some cases, the yeast extract is advantageously used in processed foods, but in most cases, the use thereof in processed foods is limited, because its peculiar flavor impairs the balance of flavors of the processed foods due to the off-flavor and off-odor resulting from its peculiar fermented odor (called yeast extract flavor).

Thus, a simple food model was developed, and each of the yeast extract and the inventive neutral flavor was added thereto, after which the sensory attributes thereof were evaluated by a panel group consisting of researchers (n=45).

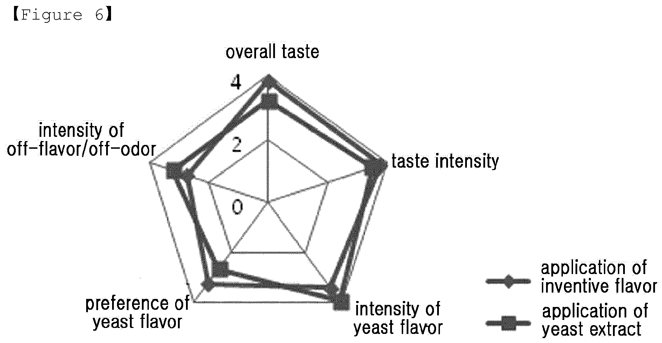

As a result, as shown in FIG. 6, in the group to which the yeast extract was applied, the intensity of yeast flavor was high, the preference to the yeast extract was low, and the tendency to recognize the yeast extract as off-flavor and off-odor appeared. Thus, it was shown that the preference of the overall taste was relatively high in the neutral flavor of the present invention.

These results suggest that off-flavor and off-odor resulting from inorganic ions were effectively removed from the neutral flavor of the present invention, and thus the neutral flavor produces a good flavor when being applied to food.

Additionally, the sensory attributes of the inventive neutral flavor and the yeast extract were compared by nine trained panels using modified-quantitative descriptive analysis. Specifically, the panels were trained for 6 hours, the sensory attributes were repeatedly measured three times, and 6 kinds of samples were simultaneously provided to the panels and randomly taken by the panels. A total of 16 attributes were evaluated in a 16-point (0-15) scale. The 16 attributes evaluated were 6 aroma attributes, 5 taste attributes, 3 flavor attributes, and 2 mouth feeling attributes. After the sample was taken, it was rinsed out with instant rice or water, and the next sample was taken. 70 ml of each sample contained in a ceramic cup was provided to the panel, and statistical analysis (Mean, MANOVA, PCA) was performed.

As a result, in the case of the natural flavor of the present invention, scorched sugar flavor, a sour smell and Solomon's seal tea flavor were strongly felt, compared to the yeast extract, and thus the natural flavor had a delicate and sour flavor. In addition, in the case of the neutral flavor, a boiled potato flavor or fragrance presumably produced from the medium component was not felt, and a boiled soy smell regarded as a fermented odor was significantly low compared to that in the yeast extract. In addition, the natural neutral flavor of the present invention had a significantly high mouth coating intensity compared to other flavors manufactured by other companies, suggesting that the natural neutral flavor can easily show a mouth coating feeling even when it is used in a small amount. Specifically, with the individual characteristics of the inventive natural neutral flavor, in connection with glutamic acid, the umami intensity was very high, and the scorched grain fragrance or flavor (Solomon's seal tea) was intense, and in connection with IMP, the umami intensity was similar to that of the yeast extract, the scorched sugar fragrance was intense, and the neutral flavor had a sour taste. In addition, it could be seen that the yeast extract had a boiled potato fragrance or flavor or a boiled soy smell.

## Claims

1. A method for preparing an inosine-5'-monophosphate (IMP) fermented broth or a glutamic acid fermented broth, comprising:
(a) fermenting a vegetable protein source with Aspergillus sp. microorganism to obtain a grain fermented broth; and
(b) fermenting a part of the grain fermented broth with Corynebacterium sp. or *Brevibacterium lactofermentum* microorganism to prepare the IMP fermented broth or glutamic acid fermented broth.

2. The method of claim 1, wherein the IMP fermented broth or the glutamic acid fermented broth are raw materials for preparing a natural flavor.

3. The method of claim 1, wherein the vegetable protein source is selected from the group consisting of soybean, corn, rice, wheat, and wheat gluten.

4. The method of claim 1, wherein the *Aspergillus sp.* microorganism is *Aspergillus sojae.*

5. The method of claim 1, wherein the Corynebacterium sp. microorganism is *Corynebacterium ammoniagenes* or *Corynebacterium glutamicum.*

6. The method of claim 1, wherein a medium composition for bacterial fermentation for preparing the IMP fermented broth or the glutamic acid fermented broth comprises a food grade material, and optionally comprises calcium panthothenate (CAPA).

7. The method of claim 1, wherein a medium for bacterial fermentation for preparing the IMP fermented broth comprises glucose, fructose, magnesium sulfate, phosphoric acid, potassium hydroxide and a grain fermented broth.

8. The method of claim 1, wherein a medium for bacterial fermentation for preparing the glutamic acid fermented broth comprises glucose, fructose, raw sugar, betaine, magnesium sulfate, potassium phosphate and phosphoric acid.

9. The method of claim 1, wherein a carbon source in a medium for bacterial fermentation for preparing the glutamic acid fermented broth is raw sugar.

10. The method of claim 1, wherein the concentration of IMP in the prepared IMP fermented broth is 50 g/L to 150 g/L, or the content of IMP in solids in the prepared IMP fermented broth is 30 wt% or more.

11. The method of claim 1, wherein the concentration of glutamic acid in the prepared glutamic acid fermented broth is 75 g/L to 150 g/L, or the content of glutamic acid in solids in the prepared glutamic acid fermented broth is 50 wt% or more.

12. The method of claim 1, further comprising (c) treating the fermented broth, obtained by the bacterial fermentation, with activated carbon; and optionally further comprising:
(i) after treating the fermented broth with activated carbon, centrifuging or filtering the treated fermented broth, or
(ii) before treating the fermented broth with activated carbon, heating the fermented broth at a temperature of 70 to 90 °C for 15 to 60 minutes to induce cell lysis.

13. A method for preparing a natural flavor, comprising:
(i) preparing the grain fermented broth, and an IMP fermented broth and a glutamic acid fermented broth by the method of claim 1; and
(ii) mixing the IMP fermented broth and glutamic acid fermented broth, and
wherein the mixing step optionally comprises a step of mixing with the grain fermented broth.

14. The method of claim 13, wherein the natural flavor is prepared using only the grain fermented broth, IMP fermented broth and glutamic acid fermented broth without adding any additional component thereto.

15. The method of claim 13, wherein the natural flavor is prepared using the grain fermented broth, IMP fermented broth and glutamic acid fermented broth without subjecting the fermented broth to an additional chemical process.

16. The method of claim 13, further comprising, before the mixing step, electrodialyzing the IMP fermented broth or the glutamic acid fermented broth.

## Patentansprüche

1. Verfahren zur Herstellung einer fermentierten Inosin-5'-Monophosphat(IMP)-Brühe oder einer fermentierten Glutaminsäurebrühe, umfassend:
(a) Fermentieren einer pflanzlichen Proteinquelle mit dem Mikroorganismus Aspergillus sp., um eine fermentierte Körnerbrühe zu erhalten; und
(b) Fermentieren eines Teils der fermentierten Körnerbrühe mit dem Mikroorganismus Corynebacterium sp. oder Brevibacterium lactofermentum, um die fermentierte IMP-Brühe oder die fermentierte Glutaminsäurebrühe herzustellen.

2. Verfahren nach Anspruch 1, wobei die fermentierte IMP-Brühe oder die fermentierte Glutaminsäurebrühe Ausgangsstoffe für die Herstellung eines natürlichen Aromas sind.

3. Verfahren nach Anspruch 1, wobei die pflanzliche Proteinquelle aus der Gruppe ausgewählt wird bestehend aus Sojabohnen, Mais, Reis, Weizen und Weizengluten.

4. Verfahren nach Anspruch 1, wobei der Mikroorganismus *Aspergillus sp. Aspergillus sojae* ist.

5. Verfahren nach Anspruch 1, wobei der Mikroorganismus *Corynebacterium sp. Corynebacterium ammoniagenes* oder *Corynebacterium glutamicum* ist.

6. Verfahren nach Anspruch 1, wobei eine Mediumzusammensetzung für die bakterielle Fermentation zur Herstellung der fermentierten IMP-Brühe oder der fermentierten Glutaminsäurebrühe einen Stoff in Nahrungsmittelqualität umfasst und optional Calciumpanthothenat (CAPA) umfasst.

7. Verfahren nach Anspruch 1, wobei ein Medium für die bakterielle Fermentation zur Herstellung der fermentierten IMP-Brühe Glucose, Fructose, Magnesiumsulfat, Phosphorsäure, Kaliumhydroxid und eine fermentierte Körnerbrühe umfasst.

8. Verfahren nach Anspruch 1, wobei ein Medium für die bakterielle Fermentation zur Herstellung der fermentierten Glutaminsäurebrühe Glucose, Fructose, Rohzucker, Betain, Magnesiumsulfat, Kaliumphosphat und Phosphorsäure umfasst.

9. Verfahren nach Anspruch 1, wobei eine Kohlenstoffquelle in einem Medium für die bakterielle Fermentation zur Herstellung der fermentierten Glutaminsäurebrühe Rohzucker ist.

10. Verfahren nach Anspruch 1, wobei die Konzentration von IMP in der hergestellten fermentierten IMP-Brühe 50 g/l bis 150 g/l beträgt, oder der Gehalt an IMP in Feststoffen in der hergestellten fermentierten IMP-Brühe 30 Gew.-% oder mehr beträgt.

11. Verfahren nach Anspruch 1, wobei die Konzentration von Glutaminsäure in der hergestellten fermentierten Glutaminsäurebrühe 75 g/l bis 150 g/l beträgt oder der Gehalt an Glutaminsäure in Feststoffen in der hergestellten fermentierten Glutaminsäurebrühe 50 Gew.-% oder mehr beträgt.

12. Verfahren nach Anspruch 1, das weiter umfasst: (c) Behandeln der fermentierten Brühe, die durch bakterielle Fermentation gewonnen wurde, mit Aktivkohle; und das optional weiter umfasst:
(i) nach dem Behandeln der fermentierten Brühe mit Aktivkohle, Zentrifugieren oder Filtern der behandelten fermentierten Brühe oder
(ii) vor dem Behandeln der fermentierten Brühe mit Aktivkohle, Erwärmen der fermentierten Brühe bei einer Temperatur von 70 bis 90 °C für 15 bis 60 Minuten, um Zelllyse zu bewirken.

13. Verfahren zur Herstellung eines natürlichen Aromas, umfassend:
(i) Herstellen der fermentierten Körnerbrühe und einer fermentierten IMP-Brühe und einer fermentierten Glutaminsäurebrühe durch das Verfahren nach Anspruch 1; und
(ii) Mischen der fermentierten IMP-Brühe und der fermentierten Glutaminsäurebrühe, und
wobei der Schritt des Mischens optional einen Schritt des Michens mit der fermentierten Körnerbrühe umfasst.

14. Verfahren nach Anspruch 13, wobei das natürliche Aroma unter Verwendung nur der fermentierten Körnerbrühe, der fermentierten IMP-Brühe und der fermentierten Glutaminsäurebrühe hergestellt wird, ohne irgendeinen zusätzlichen Bestandteil hinzuzufügen.

15. Verfahren nach Anspruch 13, wobei das natürliche Aroma unter Verwendung der fermentierten Körnerbrühe, der fermentierten IMP-Brühe und der fermentierten Glutaminsäurebrühe hergestellt wird, ohne die fermetierte Brühe einem zusätzlichen chemischen Prozess zu unterziehen.

16. Verfahren nach Anspruch 13, das weiter vor dem Schritt des Mischens das Elektrodialysieren der fermentierten IMP-Brühe oder der fermentierten Glutaminsäurebrühe umfasst.

## Revendications

1. Procédé pour préparer un bouillon fermenté d'inosine-5'-monophosphate (IMP) ou un bouillon fermenté d'acide glutamique, comprenant :
(a) la fermentation d'une source de protéine végétale avec un microorganisme Aspergillus sp. pour que soit obtenu un bouillon fermenté de grain ; et
(b) la fermentation d'une partie du bouillon fermenté de grain avec un microorganisme Corynebacterium sp. ou Brevibacterium lactofermentum pour préparer le bouillon fermenté d'IMP ou le bouillon fermenté d'acide glutamique.

2. Procédé selon la revendication 1, dans lequel le bouillon fermenté d'IMP ou le bouillon fermenté d'acide glutamique sont des matières premières pour la préparation d'une saveur naturelle.

3. Procédé selon la revendication 1, dans lequel la source de protéine végétale est choisie dans le groupe constitué par le soja, le maïs, le riz, le blé, et le gluten de blé.

4. Procédé selon la revendication 1, dans lequel le microorganisme *Aspergillus sp.* est *Aspergillus sojae.*

5. Procédé selon la revendication 1, dans lequel le microorganisme Corynebacterium sp. est *Corynebacterium ammoniagenes* ou *Corynebacterium glutamicum.*

6. Procédé selon la revendication 1, dans lequel la composition du milieu de fermentation bactérienne pour la préparation du bouillon fermenté d'IMP ou le bouillon fermenté d'acide glutamique comprend un matériau de qualité alimentaire, et comprend éventuellement du panthothénate de calcium (CAPA).

7. Procédé selon la revendication 1, dans lequel le milieu de fermentation bactérienne pour la préparation du bouillon fermenté d'IMP comprend du glucose, du fructose, du sulfate de magnésium, de l'acide phosphorique, de l'hydroxyde de potassium et un bouillon fermenté de grain.

8. Procédé selon la revendication 1, dans lequel le milieu de fermentation bactérienne pour la préparation du bouillon fermenté d'acide glutamique comprend du glucose, du fructose, du sucre non raffiné, de la bétaïne, du sulfate de magnésium, du phosphate de potassium et de l'acide phosphorique.

9. Procédé selon la revendication 1, dans lequel une source de carbone dans le milieu de fermentation bactérienne pour la préparation du bouillon fermenté d'acide glutamique est le sucre non raffiné.

10. Procédé selon la revendication 1, dans lequel la concentration d'IMP dans le bouillon fermenté d'IMP préparé est de 50 g/l à 150 g/l, ou la teneur en IMP des solides dans le bouillon fermenté d'IMP préparé est de 30 % en poids ou plus.

11. Procédé selon la revendication 1, dans lequel la concentration d'acide glutamique dans le bouillon fermenté d'acide glutamique préparé est de 75 g/l à 150 g/l, ou la teneur en acide glutamique des solides dans le bouillon fermenté d'acide glutamique préparé est de 50 % en poids ou plus.

12. Procédé selon la revendication 1, comprenant en outre (c) le traitement du bouillon fermenté, obtenu par la fermentation bactérienne, avec du charbon activé ; et comprenant éventuellement en outre :
(i) après le traitement du bouillon fermenté avec du charbon activé, la centrifugation ou la filtration du bouillon fermenté traité, ou
(ii) avant le traitement du bouillon fermenté avec du charbon activé, le chauffage du bouillon fermenté à une température de 70 à 90°C pendant 15 à 60 minutes pour induire une lyse cellulaire.

13. Procédé pour préparer une saveur naturelle, comprenant :
(i) la préparation du bouillon fermenté de grain, et d'un bouillon fermenté d'IMP et d'un bouillon fermenté d'acide glutamique par le procédé de la revendication 1 ; et
(ii) le mélange du bouillon fermenté d'IMP et du bouillon fermenté d'acide glutamique, et
dans lequel l'étape de mélange comprend éventuellement une étape de mélange avec le bouillon fermenté de grain.

14. Procédé selon la revendication 13, dans lequel la saveur naturelle est préparée par utilisation uniquement du bouillon fermenté de grain, du bouillon fermenté d'IMP et du bouillon fermenté d'acide glutamique, sans addition d'un quelconque composant additionnel à ceux-ci.

15. Procédé selon la revendication 13, dans lequel la saveur naturelle est préparée par utilisation du bouillon fermenté de grain, du bouillon fermenté d'IMP et du bouillon fermenté d'acide glutamique sans soumission du bouillon fermenté à un traitement chimique additionnel.

16. Procédé selon la revendication 13, comprenant en outre, avant l'étape de mélange, l'électrodialyse du bouillon fermenté d'IMP ou du bouillon fermenté d'acide glutamique.
